# EUROPEAN PATENT SPECIFICATION

(11) **EP 0 663 072 B1**
(45) Date of publication and mention of the grant of the patent: **05.03.1997**
(21) Application number: 93921015.9
(22) Date of filing: 28.09.1993
(51) Int. Cl.: G01N 33/72

(54) **PROCESS FOR ELIMINATING LABILE GLYCOHAEMOGLOBIN FROM A SAMPLE**
VERFAHREN ZUM ENTFERNEN VON LABILEM GLYCOHÄMOGLOBIN AUS EINER PROBE
PROCEDE D'ELIMINATION DE LA GLYCOHEMOGLOBINE LABILE D'UN ECHANTILLON

(30) Priority: 29.09.1992 GB 9220526
(43) Date of publication of application: 19.07.1995
(73) Proprietor: DREW SCIENTIFIC LTD., Barrow in Furness, Cumbria LA14 4QR (GB)
(72) Inventor: KENNEY, Andrew, Charles, Buckinghamshire SL7 1QZ (GB); ADEWUNMI, Maria, Dolapo, Manor House, London N4 1HJ (GB)
(74) Representative: Cresswell, Thomas Anthony
(86) International application number: GB9302019
(87) International publication number: WO9408242

(56) References cited:
- EP-A- 0 255 122
- EP-A- 0 271 713
- CHEMICAL ABSTRACTS, vol. 107, no. 3, 20 July 1987, Columbus, Ohio, US; abstract no. 20313, S. ODA ET AL. 'SIMPLE ELIMINATION OF LABILE GLYCOHEMOGLOBIN.' page 329 ;
- CHEMICAL ABSTRACTS, vol. 117, no. 15, 12 October 1992, Columbus, Ohio, US; abstract no. 146718, Y. HIROWATARI 'HPLC DETERMINATION OF STABLE GLYCOHEMOGLOBIN IN BLOOD.' page 421 ;
- Data for Biochemical Research (Dawson et al), third edition, Clarendon Press, Oxford, pages 421-425

## Description

The present invention relates to a process for the elimination of labile glycohaemoglobin from a sample prior to measurement of glycohaemoglobin.

The measurement of glycohaemoglobin in blood samples is widely used because its levels are indicative of longterm blood glucose concentration. Measurement of glycohaemoglobin is useful in the treatment and control of conditions where a knowledge of the blood glucose concentration is important, for example, hyperglycaemia.

Glycohaemoglobin (HbAlc) is formed by the non-enzymic reaction of glucose with haemoglobin (HbAO). The reaction proceeds in two steps. The first step is the fast, reversible formation of a Schiff's base. The second step is a slow, irreversible Amadori rearrangement leading to the HbAlc adduct. The Schiff's base intermediate (known as the "labile fraction" of HbAlc or "pre-HbAlc") is very similar chemically to stable HbAlc and it interferes with measurement of HbAlc because it is difficult to resolve the two forms by most techniques, for example, chromatography. This leads to uncertainty as to the amount of stable glycohaemoglobin present.

To prevent the labile fraction interfering with the measurement of stable HbAlc, it is necessary substantially to reduce the labile fraction to an insignificant level prior to analysis. This is usually accomplished by driving the reversible reaction, by which the Schiff's base intermediate is formed, backwards to form glucose and haemoglobin. A number of methods have previously been used to do this. These include incubation (of erythrocytes) with normal saline and incubation (of erythrocytes) at pH5 with semicarbazide/aniline.

It is known, for example, that lowering the pH leads to the elimination of labile HbAlc. However, at the values of pH at which this proceeds quickly, the haemoglobin is stable for only a short period.

It is also known that the rate of labile HbAlc elimination may be increased by heating of the sample, but this has the drawback that prolonged heating causes the sample to deteriorate. A method currently employed in the applicant's laboratory is to eliminate the labile fraction by the addition of a haemolysing solution (pH 6.0) to the sample which is then left for either two hours at room temperature (standard method) or for thirty minutes at 37°C. This has been found to minimise damage to the sample but does not completely overcome this difficulty and is inconvenient and expensive due to the prolonged reaction times.

The present invention allows labile HbAlc to be eliminated quickly and easily from a sample and is achieved using only a single reagent without substantially affecting the stability of the sample.

Accordingly, the present invention provides a process for the elimination of labile glycohaemoglobin from a sample which process comprises adding to the sample a reagent comprising a temperature dependent buffer having a pH/temperature coefficient of -0.011 or less, the reagent being added at a temperature of up to 65°C at which the buffer has a pH of less than 6.0 and reducing the temperature of the reagent thereby causing the pH to increase, the final pH being 5.5 or more. Typically, the reagent is added to the sample at a temperature of up to 56°C.

Under the conditions of low pH and elevated temperature at which the reagent is added the elimination of labile glycohaemoglobin is rapidly accomplished. Immediately after addition of the reagent, unless steps are otherwise taken, the sample begins to cool. Due to the negative temperature coefficient of the buffer, as the sample cools, the pH is raised so that sample stability is preserved.

The reagent is heated to a temperature at which the pH of the reagent is less than 6.0, preferably from 5.0 to 5.5. No additional benefit is observed when the pH is lower than 5.0 and at and above 6.0 the stability of the labile fraction is increased so that the removal rate is too slow.

The temperature at which the reagent is added is about 65°C or less, preferably 37 to 65°C, for example 37 to 56°C. At a temperature in excess of about 65°C there is a greatly increased risk that the haemoglobin will be damaged or destroyed, whereas the pH reduction at less than 37°C is small and the duration of reaction is too long. The final temperature and pH are selected with a view to the stability of the sample, particularly if it is to be stored prior to analysis. A pH of 5.5 at a temperature of 25°C or less will generally afford adequate stability, but preferably a pH of 6.0 or above is achieved.

The buffer must have a sufficiently negative pH/temperature coefficient (pKₐ/°C) to provide a low pH at the addition temperature and to revert to a pH at which the HbAlc is stable on cooling and thus has a pH/temperature coefficient of -0.011 or less, preferably -0.015 or less, in particular about -0.018.

There is no particular lower limit on the pH/temperature coefficient imposed by the process and buffers having coefficients of -0.030 or less are available.

Suitable temperature-dependant buffers are well known to those skilled in the art and are based on complex secondary or tertiary amines. Originally described by N.E. Good et al. [Biochemistry, 5, 467 (1966)], they have become known as "Good" buffers.

Examples of suitable buffers include bis(2-hydroxyethyl)imino-tris(hydroxymethyl)methane (pKₐ/°C=-0.018) ;
2-(N-morpholino) ethanesulphonic acid (pKₐ/°C=-0.011) ;
N-(2-acetamido) imino diacetic acid (pKₐ/°C=-0.011) ;
N-(2-acetamido)-2-aminoethanesulphonic acid (pKₐ/°C=-0.020) ;
2-aminoethyl-trimethylammonium chloride hydrochloride (pKₐ/°C=-0.027 as the chloride),
N,N-bis(2-hydroxyethyl)-2-aminoethanesulphonic acid (pKₐ/°C=-0.016),
N,N-bis(2-hydroxyethyl)glycine (pKₐ/°C=-0.018),
2-(cyclohexylamino) ethanesulphonic acid (pKₐ/°C=-0.011),
N-2-hydroxyethylpiperazine-N'-2-ethanesulphonic acid (pKₐ/°C=-0.014),
N-2-hydroxyethylpiperazine-N'-3-propanesulphonic acid (pKₐ/°C=-0.011),
N-glycylglycine (pKₐ/°C=-0.028),
3-(N-morpholino)propanesulphonic acid (pKₐ/°C=-0.011),
tris(hydroxymethyl)methylamine (pKₐ/°C=-0.031),
tris(hydroxymethyl)methylammonium chloride (pKₐ/°C=-0.031) N-tris(hydroxymethyl)methyl-2-aminoethanesulphonic acid (pKₐ/°C=-0.020) and
N-tris(hydroxymethyl)methylglycine (pKₐ/°C=-0.021).

By way of example, if it is desired to add the reagent at 56°C and pH 5.5 and to achieve a pH of 6.0 on cooling to 25°C, a buffer having a pH/temperature coefficient of about -0.018 is required.

Particularly preferred is bis(2-hydroxyethyl)iminotris(hydroxyethyl)methane.

Preferably the reagent used in the present invention is a haemolysing reagent containing the temperature dependant buffer, optionally also containing conventional additional haemolysing materials such as saponin, and suitable antimicrobials or preservatives such as sodium azide. In practice the haemolysing reagent will be maintained at a suitable temperature, such as 56°C, and added to samples as required. Samples will normally be aliquots of whole blood freshly taken from patients.

Elimination of the labile fraction is a complex function of temperature, incubation time and pH. The target is an elimination time of less than 15 minutes, preferably less than 10 minutes, particularly about 5 minutes, without compromising the long term stability of the sample. Shorter reaction times can be achieved by using higher temperatures, lower pH or both within the foregoing limits and by maintaining the mixture at the addition temperature then rapidly cooling rather than simply allowing the mixture to cool naturally.

The buffer, addition temperature, cooling and final temperature will be selected in order to achieve a desired duration of reaction affording sufficient elimination of the labile fraction whilst preserving the stability of the sample which may readily be determined by simple experiments.

The present invention also provides a method of measuring the concentration of stable glycohaemoglobin in a sample comprising both stable and labile fractions which comprises eliminating labile glycohaemoglobin from the sample by the process defined above and then measuring the concentration of stable glycohaemoglobin. Measurement may be by conventional techniques. Preferably the mixture is cooled to the temperature at which measurement of glycohaemoglobin concentration is to take place, for instance about 25°C. In such a case, analysis of the sample may be commenced immediately.

The final temperature and pH of the mixture will generally be selected having regard to the stability of the sample, as discussed above, and the conditions under which the HbAlc content is to be analysed. Thus, in the Example below, a Glycomat haemoglobin analyser (Ciba Corning Diagnostics Ltd) is used. This has an ion exchange column equilibrated at pH 6.2 and samples are loaded at 25°C. In this case the lowest pH which can be accommodated whilst obtaining good performance is pH 6.0. In other systems the final values of pH and temperature may be different.

Preferably the reagent is prepared and the pH adjusted to take into account the conditions of temperature and pH at which measurement of the glycohaemoglobin is to take place. The pH may be adjusted by the addition of a suitable acid, for example concentrated hydrochloric acid, so that the pH at the temperature at which analysis takes place is suitably controlled. If pH adjustment is undertaken at any other temperature, the target pH value may be calculated by use the pH/temperature coefficient of the buffer. For example, in the Example below, the pH of the haemolysing reagent is adjusted so that at 25°C a pH of 6.0 will be achieved. For this, accurate measurement of temperature is required.

The following Examples illustrate the invention but are not intended to limit the extent of protection.

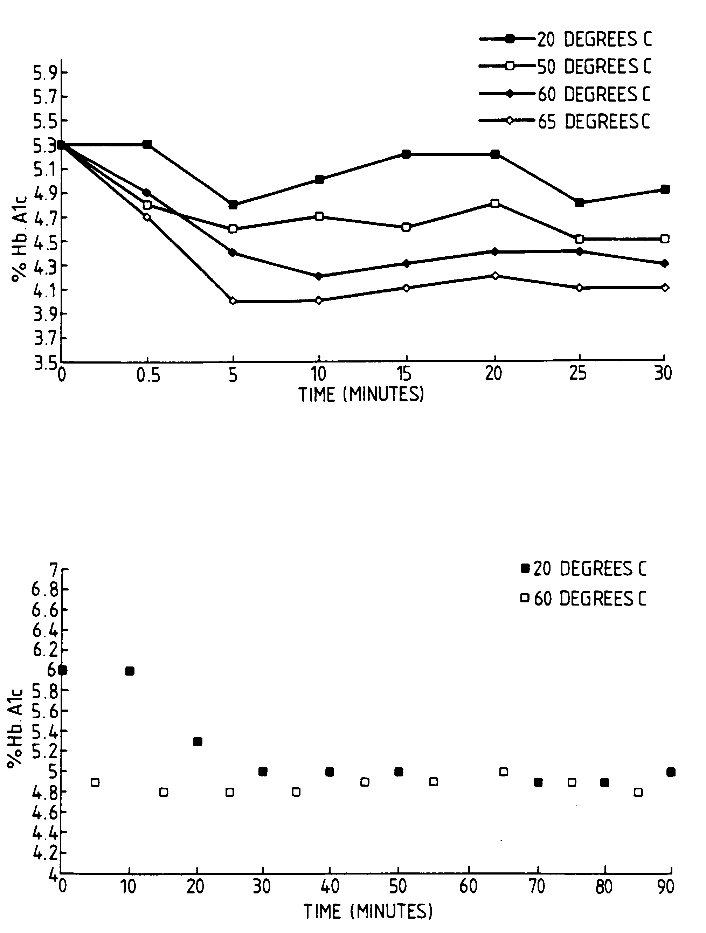

In Figures 1 to 5 referred to in the Examples % HbAlc refers to the concentration of measurable glycohaemoglobin of stable and, where present, liable glycohaemoglobin.

### EXAMPLE 1

### Rate of labile fraction removal at 56°C

Bis(2-hydroxyethyl)imino-tris(hydroxymethyl)methane, (0.836g, 40 mM, Sigma Chemical Co.), Saponin from Gypsophilia sp. (5 mg, Sigma Chemical Co.) and sodium azide (20mg, Sigma Chemical Co.) were dissolved in deionised water (80 ml) and titrated with concentrated hydrochloric acid (Fisons) so that the pH at 25°C was 6.0. Deionised water was added to make the volume up to 100 ml. The solution was left to stand for 2 hours after which the pH was readjusted. The haemolysing reagent was then heated to the experimental temperature (56°C).

Samples (20 µl) of fresh human blood anticoagulated with ethyl diamine tetracetic acid (EDTA) were pipetted into microcentrifuge tubes (Glycomat sample vials in a Glycomat Autoloader rack).

Haemolysis and labile fraction elimination were initiated by adding heated haemolysing reagent (1 ml) to the samples using a dispenser pipette. Following addition of the heated haemolysing reagent the blood samples were allowed to cool to room temperature. Analysis for HbAlc took place at approximately 5 minutes after addition of the haemolysing reagent using a Glycomat haemoglobin analyzer (Ciba Corning Diagnostics Ltd) equipped with Glycomat Blue Fast HbAlc reagent kits (Ciba Corning Diagnostics Ltd.) for 5 minute HbA1 (labile)/HbAlc assays according to the manufacturers instructions.

The amount of glycohaemoglobin in the samples was about 6% of total haemoglobin and did not change significantly when retested at 5 minute intervals over 100 minutes showing that the content of labile fraction is rapidly and substantially reduced at 56°C.

### Example 2

### Rate of labile fraction removal at 20, 50, 60 and 65°C

Example 1 was repeated for haemolysing reagent temperatures of 20, 50, 60 and 65°C. The amount of glycohaemoglobin in the samples was measured over a 30 minute period. The results obtained are expressed graphically in Figure 1.

Figure 1 shows that increasing the temperature at which the haemolysing reagent is added increases the rate of removal of labile glycohaemoglobin. The most rapid reduction in the percentage of measurable glycohaemoglobin (and thus the most rapid elimination of the labile fraction) between 0 and 5 minutes occurs at a haemolysing reagent temperature of 65°C.

The figure also shows that for a haemolysing reagent temperature of 65°C the amount of measurable glycohaemoglobin remains effectively constant at times in excess of 5 minutes. This means that at this temperature all of the labile glycohaemoglobin is eliminated from the sample in under 5 minutes.

### Example 3

### Removal of glycated haemoglobin at 20 and 60°C

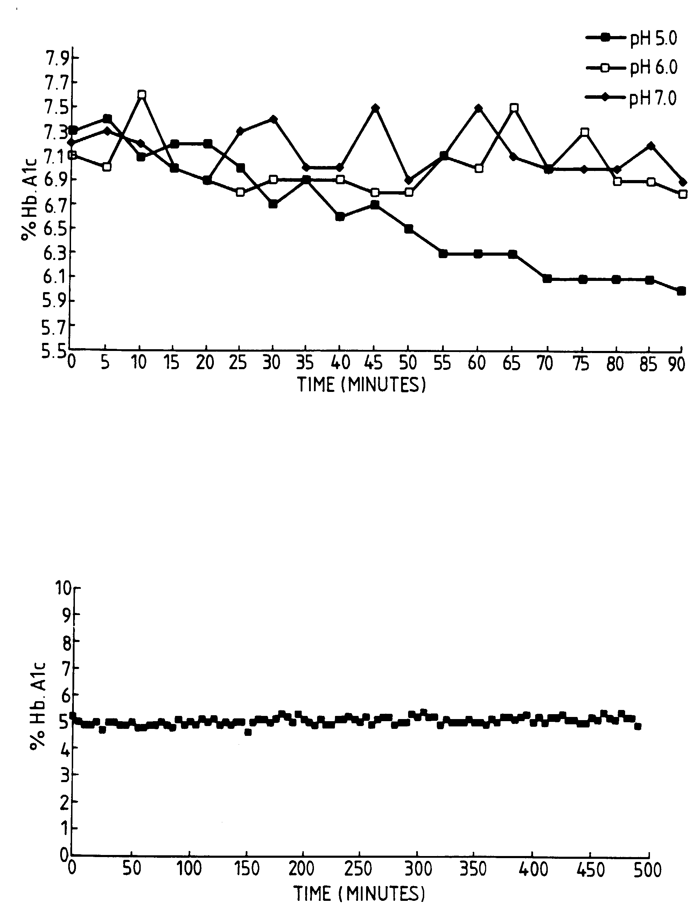

Example 1 is repeated for haemolysing reagent temperatures of 20 and 60°C over a period of 90 minutes. The results obtained are shown in Figure 2.

At 60°C the removal of the labile glycohaemoglobin fraction occurs much more rapidly than at ambient temperature (20°C). The percentage of measurable glycohaemoglobin reached an equilibrium value after only about 5 minutes for a haemolysing reagent temperature of 60°C, whereas at 20°C the equilibrium value was not reached until in excess of 60 minutes. This equilibrium value corresponds to the proportion of stable glycohaemoglobin in the sample, the labile fraction having been eliminated.

### Example 4

### Removal of labile glycated haemoglobin at pH5, 6 and 7

Bis(2-hydroxyethyl)imino-tris(hydroxymethyl)methane, (0.836,g Sigma Chemical Co.), Saponin from Gypsophilia sp. (5 mg, Sigma Chemical Co.) and sodium azide (20 mg, Sigma Chemical Co.) were dissolved in deionised water (80 ml) and titrated with concentrated hydrochloric acid (Fisons) to the appropriate pH (5.0, 6.0 or 7.0) at 25°C. Deionised water was added to make the volume up to 100 ml. The solution was left to stand for 2 hours after which the pH was readjusted.

Samples (20 µl) of fresh human blood anticoagulated with ethyl diamine tetracetic acid (EDTA) were pipetted into microcentrifuge tubes (Glycomat sample vials in a Glycomat Autoloader rack).

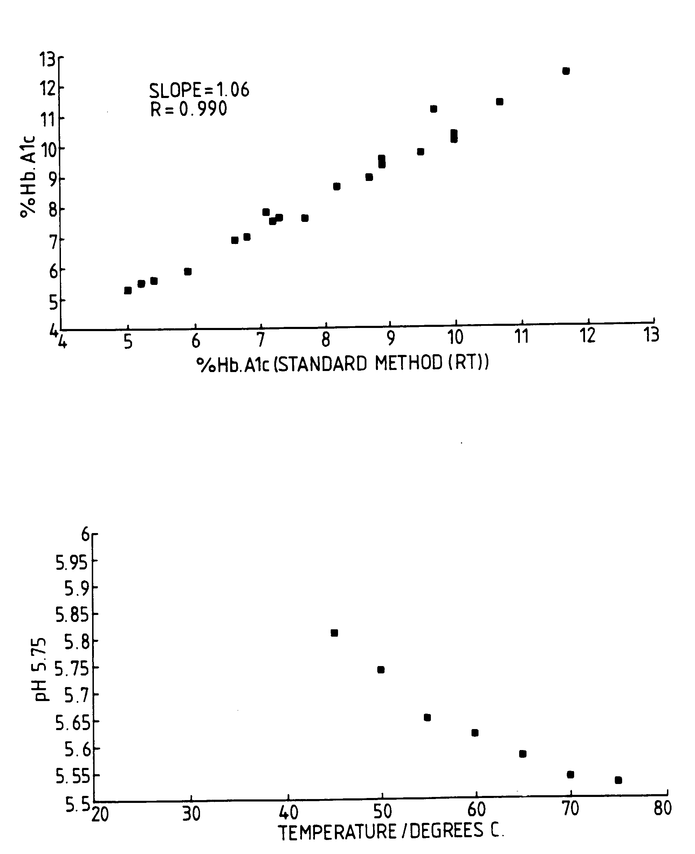

The haemolysing reagent is added to the blood samples at 25°C and then analysed for HbAlc at approximately 5 minutes thereafter by the same procedure as in Example 1. The results are shown in Figure 3.

At ambient temperature the time taken to a reach the equilibrium value of measurable glycohaemoglobin increases with increasing pH. As it is known that elimination of labile glycohaemoglobin proceeds more rapidly at low pH it can be concluded that rapid removal of labile glycohaemoglobin can only be achieved using a combination of low pH (below 6.0) and high temperature.

### Example 5

### Stability of Sample glycohaemoglobin with time

The procedure of Example 1 was repeated using a haemolysing reagent temperature of 65°C corresponding to a pH of approximately 5.3. Measurement of glycohaemoglobin concentration took place over a period of 500 minutes. The results obtained are shown in Figure 4.

The data in the figure confirm that the amount of measurable glycohaemoglobin remains constant even when the sample is exposed to extremes of pH and temperature. The method of the invention exposes the sample to these conditions only transiently and this has the advantage of preserving sample integrity.

### Example 6

### Comparison of the method of the invention with standard method

A regression analysis is performed on data obtained using the standard method referred to earlier (a haemolysing solution of pH 6.0 at room temperature for two hours) and data obtained using the procedure of Example 1 for a haemolysing reagent temperature of 65°C. The results are shown in Figure 5.

The figure is a plot of equilibrium glycohaemoglobin concentration obtained using the standard method versus the equilibrium glycohaemoglobin concentration obtained by the present method for a number of samples having different initial glycohaemoglobin (stable plus labile) concentrations.

The correlation coefficient, R, is equal to 0.990 showing that methods correlate well, and can therefore be considered equivalent in terms of data accuracy.

### Example 7

### Effect of temperature on haemolysing reagent pH

The effect of temperature on the pH of the haemolysing reagent of Example 1 is investigated. The results are shown in Figure 6.

Increasing the temperature of the haemolysing reagent is accompanied by a reduction in the pH. The type of plot shown in Figure 6 may be used as a calibration curve for a given buffer contained in the haemolysing reagent to identify what temperature should be employed to obtain a particular pH.

## Claims

1. A process for the elimination of labile glycohaemoglobin from a sample which process comprises adding to the sample a reagent comprising a temperature dependent buffer having a pH/temperature coefficient of -0.011 or less, the reagent being added at a temperature of up to 65°C at which the buffer has a pH of less than 6.0 and reducing the temperature of the reagent thereby causing the pH to increase, the final pH being 5.5 or more.

2. A process according to claim 1, wherein the temperature at which the reagent is added is from 37 to 65°C

3. A process according to claim 1 or 2, wherein the pH at which the reagent is added is from 5.0 to 5.5.

4. A process according to claim 1, 2 or 3, wherein the temperature of the reagent is reduced such that the pH increases to 6.0 or more.

5. A process according to any one of the preceding claims, wherein the temperature dependent buffer is:
bis(2-hydroxyethyl)imino-tris(hydroxymethyl)methane;
2-(N-morpholino)ethanesulphonic acid;
N-(2-acetamido)imino diacetic acid;
N-(2-acetamido)-2-aminoethanesulphonic acid;
2-aminoethyl-trimethylammonium chloride hydrochloride;
N,N-bis(2-hydroxyethyl)-2-aminoethanesulphonic acid;
N,N-bis(2-hydroxyethyl)glycine;
2-(cyclohexylamino)ethanesulphonic acid;
N-2-hydroxyethylpiperazine-N'-2-ethanesulphonic acid;
N-2-hydroxyethylpiperazine-N'-3-propanesulphonic acid;
N-glycylglycine;
3-(N-morpholino)propanesulphonic acid;
tris(hydroxymethyl)methylamine;
tris(hydroxymethyl)methylammonium chloride;
N-tris(hydroxymethyl)methyl-2-aminoethanesulphonic acid; and
N-tris(hydroxymethyl)methylglycine.

6. A process according to any one of the preceding claims, wherein the temperature dependent buffer has a pH/temperature coefficient of -0.015 or less.

7. A process according to claim 6, wherein the temperature dependent buffer has a pH/temperature coefficient of -0.018.

8. A process according to claim 7, wherein the temperature dependent buffer is bis(2-hydroxyethyl)iminotris(hydroxymethyl)methane.

9. A process according to any one of the preceding claims, wherein the reagent is incubated with the sample for less than 15 minutes.

10. A process according to claim 9, wherein the reagent is incubated with the sample for less than 10 minutes.

11. A process according to claim 10, wherein the reagent is incubated with the sample for about 5 minutes.

12. A process according to any one of the preceding claims, wherein the reagent is a haemolysing reagent containing the temperature dependent buffer.

13. A process according to claim 12, wherein the reagent further contains saponin.

14. A process according to claim 13, wherein the reagent further contains an antimicrobial agent or preservative.

15. A method of measuring the concentration of stable glycohaemoglobin in a sample comprising both stable and labile fractions, which comprises eliminating labile glycohaemoglobin from the sample by the process according to any one of claims 1 to 14 and then measuring the concentration of stable glycohaemoglobin.

## Patentansprüche

1. Verfahren zur Entfernung von labilem Glykohämoglobin aus einer Probe, welches Verfahren die Zugabe zu einer Probe eines Reagenz, das einen Temperatur-abhängigen Puffer mit einem pH/Temperaturkoeffizient von -0,011 oder weniger enthält, wobei das Reagenz bei einer Temperatur von bis zu 65°C zugegeben wird, bei der der Puffer einen pH-Wert von weniger als 6,0 aufweist, und die Reduzierung der Temperatur des Reagenz umfaßt, wobei der pH-Wert zum Steigen gebracht wird, so daß der pH-Endwert 5,5 oder mehr beträgt.

2. Verfahren nach Anspruch 1, worin die Temperatur, bei der das Reagenz zugegeben wird, von 37 bis 65°C beträgt.

3. Verfahren nach Anspruch 1 oder 2, worin der pH-Wert, bei dem das Reagenz zugegeben wird, von 5,0 bis 5,5 beträgt.

4. Verfahren nach Anspruch 1, 2 oder 3, worin die Temperatur des Reagenz derart reduziert wird, daß der pH-Wert auf 6,0 oder mehr steigt.

5. Verfahren nach einem der vorangegangenen Ansprüche, worin der Temperatur-abhängige Puffer ist:
Bis(2-hydroxyethyl)imino-tris(hydroxymethyl)methan; 2-(N-Morpholino)ethansulfonsäure;
N-(2-Acetamido)iminadiessigsäure;
N-(2-Acetamido)-2-aminoethansulfonsäure;
2-Aminoethyl-trimethylammoniumchlorid-Hydrochlorid;
N,N-Bis(2-hydroxyethyl)-2-aminoethansulfonsäure;
N,N-Bis(2-hydroxyethyl)glycin;
2-(Cyclohexylamino)ethansulfonsäure;
N-2-Hydroxyethylpiperazin-N'-2-ethansulfonsäure;
N-2-Hydroxyethylpiperazin-N'-3-propansulfonsäure;
N-Glycylglycin:
3-(N-Morpholin)propansulfonsäure;
Tris(hydroxymethyl)methylamin;
Tris(hydroxymethyl)methylammoniumchlorid;
N-Tris(hydroxymethyl)methyl-2-aminoethansulfonsäure; und
N-Tris(hydroxymethyl)methylglycin.

6. Verfahren nach einem der vorangegangenen Ansprüche, worin der Temperatur-abhängige Puffer einen pH/Temperaturkoeffizient von -0,015 oder weniger aufweist.

7. Verfahren nach Anspruch 6, worin der Temperatur-abhängige Puffer einen pH/Temperaturkoeffizient von -0,018 aufweist.

8. Verfahren nach Anspruch 7, worin der Temperatur-abhängige Puffer Bis(2-hydroxyethyl)imino-tris(hydroxymethyl)methan ist.

9. Verfahren nach einem der vorangegangenen Ansprüche, worin das Reagenz mit der Probe weniger als 15 Minuten lang inkubiert wird.

10. Verfahren nach Anspruch 9, worin das Reagenz mit der Probe weniger als 10 Minuten lang inkubiert wird.

11. Verfahren nach Anspruch 10, worin das Reagenz mit der Probe weniger als 5 Minuten lang inkubiert wird.

12. Verfahren nach einem der vorangegangenen Ansprüche, worin das Reagenz ein hämolysierendes Reagenz ist, das den Temperatur-abhängigen Puffer enthält.

13. Verfahren nach Anspruch 12, worin das Reagenz ferner Saponin enthält.

14. Verfahren nach Anspruch 13, worin das Reagenz ferner ein antimikrobielles Mittel oder Konservierungsmittel enthält.

15. Verfahren zur Messung der Konzentration von stabilem Glykohämoglobin in einer Probe, umfassend sowohl stabile als auch labile Fraktionen, welches das Entfernen von labilem Glykohämoglobin aus der Probe mittels des Verfahrens nach einem der Ansprüche 1 bis 14 und dann das Messen der Konzentration von stabilem Glykohämoglobin umfaßt.

## Revendications

1. Procédé pour l'élimination de la glycohémoglobine labile d'un échantillon, ce procédé comprenant les étapes consistant à ajouter à l'échantillon un réactif comprenant un tampon dépendant de la température ayant un coefficient pH/température de -0,011 ou moins, le réactif étant ajouté à une température allant jusqu'à 65 °C à laquelle le tampon a un pH inférieur à 6,0 et à réduire la température du réactif, provoquant ainsi l'augmentation du pH, le pH final étant de 5,5 ou plus.

2. Procédé selon la revendication 1, dans lequel la température à laquelle le réactif est ajouté est de 37 à 65 °C.

3. Procédé selon la revendication 1 ou 2, dans lequel le pH auquel le réactif est ajouté est de 5,0 à 5,5.

4. Procédé selon la revendication 1, 2 ou 3, dans lequel la température du réactif est réduite de sorte que le pH augmente à 6,0 ou plus.

5. Procédé selon l'une quelconque des revendications précédentes, dans lequel le tampon dépendant de la température est :
le bis(2-hydroxyéthyl)imino-tris(hydroxyméthyl)-méthane ;
l'acide 2-(N-morpholino)éthanesulfonique;
l'acide N-(2-acétamido)iminodiacétique;
l'acide N-(2-acétamido)-2-aminoéthanesulfonique ;
le chlorhydrate de chlorure de 2-aminoéthyltriméthylammonium ;
l'acide N,N-bis(2-hydroxyéthyl)-2-aminoéthanesulfonique ;
la N,N-bis(2-hydroxyéthyl)glycine ;
l'acide 2-(cyclohexylamino)éthanesulfonique ;
l'acide N-2-hydroxyéthylpipérazine-N'-2-éthanesulfonique ;
l'acide N-2-hydroxyéthylpipérazine-N'-3-propanesulfonique ;
la N-glycylglycine ;
l'acide 3-(N-morpholino)propanesulfonique ;
la tris(hydroxyméthyl)méthylamine ;
le chlorure de tris(hydroxyméthyl)méthylammonium ;
l'acide N-tris(hydroxyméthyl)méthyl-2-aminoéthanesulfonique ; et
la N-tris(hydroxyméthyl)méthylglycine.

6. Procédé selon l'une quelconque des revendications précédentes, dans lequel le tampon dépendant de la température a un coefficient pH/température de -0,015 ou moins.

7. Procédé selon la revendication 6, dans lequel le tampon dépendant de la température a un coefficient pH/température de -0,018.

8. Procédé selon la revendication 7, dans lequel le tampon dépendant de la température est le bis(2-hydroxyéthyl)imino-tris(hydroxyméthyl)méthane.

9. Procédé selon l'une quelconque des revendications précédentes, dans lequel le réactif est incubé avec l'échantillon pendant moins de 15 minutes.

10. Procédé selon la revendication 9, dans lequel le réactif est incubé avec l'échantillon pendant moins de 10 minutes.

11. Procédé selon la revendication 10, dans lequel le réactif est incubé avec l'échantillon pendant environ 5 minutes.

12. Procédé selon l'une quelconque des revendications précédentes, dans lequel le réactif est un réactif hémolysant contenant le tampon dépendant de la température.

13. Procédé selon la revendication 12, dans lequel le réactif contient en outre de la saponine.

14. Procédé selon la revendication 13, dans lequel le réactif contient en outre un agent antimicrobien ou un conservateur.

15. Procédé de mesure de la concentration de glycohémoglobine stable dans un échantillon comprenant tant des fractions stables que des fractions labiles, qui comprend les étapes consistant à éliminer la glycohémoglobine labile de l'échantillon par le procédé selon l'une quelconque des revendications 1 à 14 puis à mesurer la concentration de glycohémoglobine stable.
